Europäisches Patentamt

European Patent Office

Office européen des brevets

(19)

(11) Veröffentlichungsnummer: **0 105 401**

**A2**

# EUROPÄISCHE PATENTANMELDUNG

(12)

(21) Anmeldenummer: 83109303.4

(22) Anmeldetag: 20.09.83

(51) Int. Cl.³: **G 01 N 21/89**
**G 01 B 11/30**

(30) Priorität: 01.10.82 DE 3236416

(43) Veröffentlichungstag der Anmeldung:
18.04.84 Patentblatt 84/16

(84) Benannte Vertragsstaaten:
AT BE CH DE FR GB IT LI LU NL SE

(71) Anmelder: Licentia Patent-Verwaltungs-GmbH
Theodor-Stern-Kai 1
D-6000 Frankfurt/Main 70(DE)

(71) Anmelder: MANNESMANN Aktiengesellschaft
Mannesmannufer 2
D-4000 Düsseldorf 1(DE)

(72) Erfinder: Schöne, Gerhard, Dipl.-Ing.
Rissener Strasse 82
D-2000 Wedel(DE)

(72) Erfinder: Aurin, Ingo, Dipl.-Ing.
Friedhofsweg 38c
D-2081 Holm(DE)

(72) Erfinder: Ehlert, KLaus-Peter, Dr. Ing.
Käthe-Kollwitz-Strasse 33
D-4130 Moers 3(DE)

(72) Erfinder: Meyer, Karl-Heinz
Walderbenweg 38
D-4100 Duisburg 29(DE)

(72) Erfinder: Deppe, Gerd-Joachim, Dr. Ing.
Dürerstrasse 17
D-4100 Duisburg(DE)

(72) Erfinder: Geisbüsch, Peter, Dipl.-Ing.
Am Hohledey 24
D-4030 Ratingen(DE)

(74) Vertreter: Sass, Adolph, Dipl.-Ing. et al,
Licentia Patent-Verwaltungs-GmbH Theodor-Stern-Kai 1
D-6000 Frankfurt 70(DE)

(54) Verfahren und Vorrichtung zur Qualitätsbeurteilung von Stahloberflächen.

(57) Verfahren und Vorrichtung zur Qualitätsbeurteilung von Stahloberflächen, insbesondere zur Erkennung von Oberflächenfehlern, wie Rissen an heißen entzunderten Stranggußprodukten. In einer Stranggußmaschine ist ein optronischer Sensor, z.B. eine elektronische Kamera mit elektronischer Kurzzeitbelichtung angeordnet, an dem die zu kontrollierenden Produkte vorbeigeführt werden. Der optronische Sensor empfängt die von den Produkten emittierte Oberflächenstrahlung und setzt diese in auswertbare Signale um. Dem Sensor ist eine Verarbeitungseinrichtung zur automatischen Erkennung und Auswertung der Oberflächenfehler nachgeschaltet. Die vorzugsweise digitale Verarbeitungseinrichtung wird nach vorgegebenen Längen- und Breitenkriterien der Oberflächenfehler zur Qualitätsbeurteilung herangezogen.

FIG.2

EP 0 105 401 A2

Licentia Patent-Verwaltungs-GmbH          PTL-HH/Sl/mar
Theodor-Stern-Kai 1                        HH 82/22
D-6000 Frankfurt 70

und

MANNESMANN AKTIENGESELLSCHAFT
Mannesmannufer 2
D-4000 Düsseldorf 1

"Verfahren und Vorrichtung zur Qualitätsbeurteilung von Stahloberflächen"

Die Erfindung betrifft ein Verfahren zur Qualitätsbeurteilung
von Stahloberflächen, insbesondere zur Erkennung von Oberflächenfehlern, wie Rissen an heißen, entzunderten Stranggußprodukten,
wobei in einer Stranggußmaschine ein optronischer Sensor angeordnet ist, an dem die Produkte vorbeigeführt werden, der die
von den Produkten emittierte Oberflächenstrahlung empfängt und
in auswertbare Signale umsetzt, und der mit einer nachgeschalteten Verarbeitungseinrichtung zur automatischen Erkennung und
Auswertung der Oberflächenfehler verwendet wird.

Beim Stranggießen von Stahl müssen u.a. bestimmte Abkühlbedingungen eingehalten werden, weil sich sonst am Produkt Risse
bilden, die, wenn sie eine bestimmte Größe überschreiten, das
Werkstück für die sofortige Weiterverarbeitung unbrauchbar machen. Bisher konnten Risse nur am kalten Werkstück visuell festgestellt werden, so daß zwischen der Entstehung des Fehlers und
seiner Erkennung eine relativ lange Zeitspanne vergangen war und
die Fehlerursache erst abgestellt werden konnte, nachdem bereits
eine größere Menge fehlerhaftes Material angefallen war. Man
hat daher versucht, die Inspektion auf Fehler bereits am heißen

Produkt durchzuführen. Da Oberflächenfehler bei Temperaturen zwischen 700 und $1100^{\circ}C$, wie sie der durcherstarrte Strang am Ausgang der Stranggießmaschine aufweist, ohne zusätzliche Bearbeitung vom menschlichen Auge nicht festzustellen sind, wurde eine Dioden-Zeilen-Kamera eingesetzt, die auf einer in sich geschlossenen Bahn ständig um den Strang bewegt wird. Die Fehler der selbststrahlenden Strangoberfläche werden von der Kamera zeilenweise als Intensitätsschwankungen erfasst und einer Steuer- und Auswerteelektronik zugeführt.

Es hat sich gezeigt, daß die bekannte Einrichtung nicht ausreicht, um Fehler in der heißen Oberfläche aufzufinden, sondern daß die Beobachtung des Werstücks unter bestimmten zusätzlichen Gegebenheiten erfolgen muß. Es wurde nun gefunden, daß es gelingt, an heißen Stranggußprodukten die Oberflächenqualität zuverlässig zu beurteilen, wenn das Produkt an einer elektronischen Kamera mit elektronischer Kurzzeitbelichtung vorbeibewegt wird, und wenn eine digitale Verarbeitungseinrichtung nach vorgegebenen Längen- und Breitenkriterien der Oberflächenfehler zur Qualitätsbeurteilung herangezogen wird.

Das eingangs angedeutete Entzundern kann mit Wasser erfolgen, das unter sehr hohem Druck (ca. 200 bar) auf das Werkstück gespritzt wird. Das Wasser muß entsprechend aufbereitet sein, damit sich die Düsen nicht verstopfen oder durch Ablagerungen zusetzen. Um die gewünschte Schälwirkung zu erzielen, muß der Abstand zwischen der Austrittsöffnung der Düsen und der Werkstückoberfläche kurz sein, wobei dafür gesorgt wird, daß das Wasser schräg auf das Werkstück auftrifft.

Mit dem erfindungsgemäßen Verfahren gelingt es, Risse, die am zwischen 700 und $1100^{\circ}C$ heißen Stranggießstück als Strukturen auftreten, die mit einem Kontrast von 1 % und größer als die umgebende Oberfläche strahlen, automatisch zu erkennen und zur Bildverarbeitung zu benutzen.

Dadurch wird wiederum eine entscheidende Verbesserung der
Wirtschaftlichkeit des Betriebs, der Stranggußprodukte weiterverarbeitet, erzielt, weil die bisher zur Oberflächenbeurteilung erforderliche Abkühlung und damit auch die zur Weiterverarbeitung erforderliche erneute Erwärmung überflüssig wird.
Wenn die Stranggußprodukte durch Walzen weiterverarbeitet
werden sollen, so kann die thermische Energie der Stranggießprodukte für die Weiterverarbeitung genutzt werden. Das

- 3 -

Walzwerk kann dadurch unmittelbar an das Stahlwerk angekoppelt werden. Die Werkstücke, die die Qualitätsanforderungen nicht erfüllen, können in heißem Zustand ausgesondert werden. Vorteile erbringt das erfindungsgemäße Verfahren auch in den Fällen, in denen schadhafte Oberflächen nachbearbeitet werden, z.B. durch Flämmen. Werden mit Hilfe des erfindungsgemäßen Verfahrens die Oberflächenteile, deren Qualität hinter dem vorgegebenen Standart zurückbleibt, ermittelt, so daß die Nacharbeit nur an den beanstandeten Stellen vorgenommen werden kann, ergeben sich ebenfalls erhebliche wirtschaftliche Vorteile. So war es z.B. bisher erforderlich, Werkstücke, an denen durch Flämmen zu beseitigende Fehler auftraten, insgesamt zu flämmen, weil die eine Nacharbeit erfordernden Stellen am heißen Werkstück nicht ermittelt werden konnten. Vor allem verursacht das Flämmen einen Materialverlust von bis zu 3 % des Werkstückgewichts, so daß sich eine Verbesserung dieses Arbeitsschrittes besonders stark auf die Wirtschftlichkeit auswirkt.

Ausgestaltungen des erfindungsgemäßen Verfahrens sind in den Unteransprüchen 2 bis 4 beschrieben.

Eine erfindungsgemäße Vorrichtung zur Durchführung des Verfahrens ist in Anspruch 5 beansprucht.

Weiterbildungen der erfindungsgemäßen Vorrichtung sind aus den Unteransprüchen 6 bis 13 zu entnehmen.

Ein besonderer Vorteil der Erfindung ist darin zu sehen, daß die Oberflächenfehler an den heißen Stranggußprodukten schon in der Inspektionsstrecke einer Stranggußmaschine erkannt werden. Es ist somit vorteilhafterweise möglich, mit Oberflächenfehlern behaftete Produkte schnell zu klassifizieren bzw. im heißen Zustand einem Glühofen zuzuführen. Ein weiterer Vorteil der Erfindung besteht in einer einfachen Markierungsmöglichkeit der erfaßten Oberflächenfehler, z.B. mittels einer Farbspritzpistole.

In der Zeichnung ist ein Ausführungsbeispiel nach der Erfindung dargestellt, u.z. zeigt:

Fig. 1 einen Ausschnitt eines Stranggußknüppels mit mehreren
Rissen als Oberflächenfehler,

Fig. 2 ein Blockschaltbild einer Vorrichtung zur Erkennung
und Auswertung von Oberflächenfehlern, und

Fig. 3 den Verfahrensablauf in einer Stranggußmaschine, die
mit einer Vorrichtung gemäß Figur 2 ausgerüstet ist.

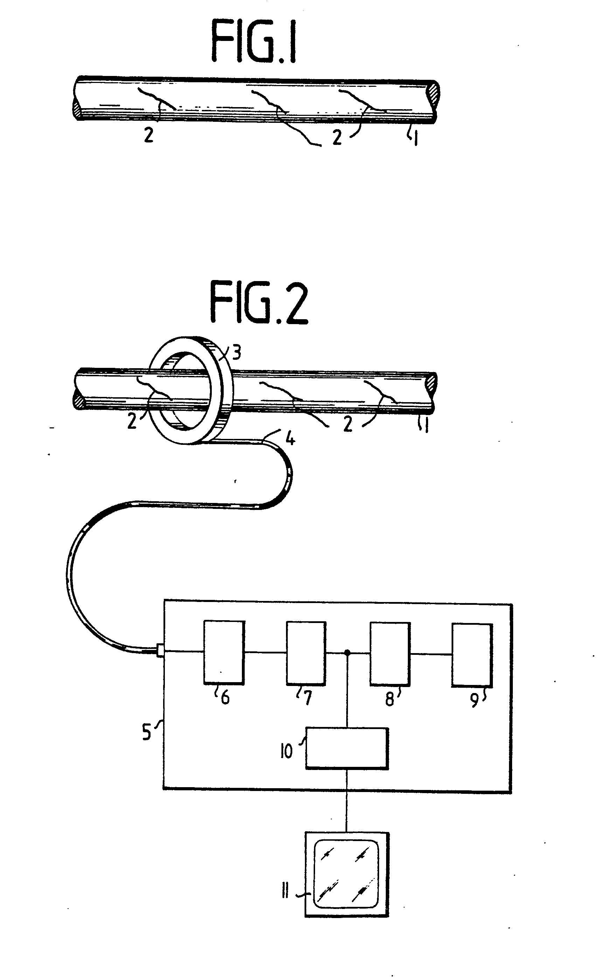

In Figur 1 besitzt ein Stranggußknüppel 1 auf seiner Oberfläche
Risse 2, wobei der Stranggußknüppel 1 eine Oberflächentemperatur
von etwa 800° C aufweist und mit einer Geschwindigkeit von beispielsweise 1 m/s in einer Stranggußmaschine fortbewegt wird.
Der Riß, der etwa 1 mm breit, 20 mm lang und schlangenlinienförmig ist, soll vorzugsweise im heißen Zustand des Knüppels
1 erkannt und ausgewertet werden.

Die in Figur 2 dargestellte Vorrichtung weist einen optoelektronischen Sensor 3 auf, der ringförmig ausgebildet ist und einen
heißen Knüppel 1 umgibt. Der Durchmesser des Sensors 3 ist so
groß gewählt, daß zwischen dem Innenring des Sensors 3 und
der Oberfläche des Knüppels 1 ein ausreichender, eine Beschädigung des Sensors durch zu hohe Temperaturen vermeidender Abstand besteht. Der Ausgang des Sensors 3 ist über ein Kabel
4 an eine Elektronikbox 5 angeschlossen, die eine Einrichtung
6 zur Ansteuerung und Harmonisierung der unten beschriebenen
Sensorelemente des Sensors 3, eine Binärisierungseinrichtung 7,
eine Einrichtung 8 zur Erkennung von Oberflächenfehlern 2 und
eine Einrichtung 9 zur Lagevermessung und Klassifizierung von
Oberflächenfehlern 2 sowie zur Alarmgebung bei Auftritt von
Oberflächenfehlern 2 aufweist.

Die Elektronikbox 5 enthält weiterhin eine Einrichtung zur
Spannungsversorgung des Sensors 3 bzw. der Sensorelemente und
eine Filtereinrichtung zur Unterdrückung von impulsförmigen
Störungen auf der Netzleitung, wobei diese Einrichtungen
zeichnerisch nicht dargestellt sind. Zwischen der Binärisierungseinrichtung 7 und der Einrichtung 8 zur Erkennung von Oberflächenfehlern ist eine Bildspeichereinrichtung 10 angeschlossen, deren Ausgang mit einem außerhalb der Elektronikbox 5 angeordneten Monitor 11 in elektrisch leitender Verbindung steht. Die Einrichtungen 8 bis 10 stellen praktisch die Bildverarbeitungseinrichtung dar. Von der Elektronikbox 5 zum Sensor 3 werden die Versorgungsspannungen und die Mutterfrequenz übertragen, umgekehrt ein analoges Sensorsignal sowie ein Umlaufsynchronsignal.

Der optronische Sensor 3 weist eine vorgegebene Anzahl von zeichnerisch nicht dargestellten Sensorelementen auf, die beispielsweise in einem Aluminiumring angeordnet und auf dem Umfang des Ringes gleichmässig verteilt sind. Jedes Sensorelement tastet einen bestimmten Sektor des Knüppels 1 ab, der durch den Sensor 3 durchgeschoben wird. Der Sensor 3 gibt die von den Sensorelementen erzeugten Bildsignale über die Einrichtung 6 zur Harmonisierung an die Bildverarbeitungseinrichtung 7 bis 10 ab.

Die Form des Sensors 3 ist zweckmässigerweise der Produktform angepasst. So wird beispielsweise für Stranggußbrammen ein rechteckförmiger Sensor benötigt, bei dem die einzelnen Sensorelemente auf die Sensorseiten verteilt sind. Als Sensor bzw. Sensorelemente kommen verschiedene Aufnahmegeräte in Frage. Zum Beispiel kann eine CCD-Zeilen-Sensorkombination (charge-coupled-device-Zeilen-Sensorkombination), eine CCD-Matrix-Sensorkonfiguration, eine Kurzzeitbelichtungskamera auf Röhren-Sensorbasis oder eine Kurzzeitbelichtungskamera auf CCD-Matrix-Sensorbasis verwendet werden.

Die Anzahl der Sensorelemente ist durch den Durchmesser der Knüppel 1, die Arbeitsentfernung zwischen den Sensorelementen und der Auflösung der Sensorelemente bestimmt und beträgt im vorliegenden Beispiel 16. Auch kann jedes Sensorelement zur Abtastung seines zugehörenden Sektors aus einer aus 256 Elementen bestehenden Fotodiodenzeile bestehen. Es ist dann ein zeichnerisch nicht dargestellter Taktgeber vorhanden, der die Sensorelemente zyklisch derart ansteuert, daß pro Umlauf ein serielles Signal aus einer bestimmten Anzahl von Bildpunkten besteht. Die Anzahl der Bildpunkte ergibt sich durch Multiplikation der 16 Sensorelemente mit den 256 Elementen und ist demnach 4096. Für Knüppel 1 mit einem Durchmesser von 200 mm und einem Umfang von $\pi$ x 200 mm und 4096 Bildpunkten ergibt sich ein Bildpunktabstand von ca. 0,15 mm, der kleinen Rißbreiten adäquat ist. Beträgt die durch den Taktgeber vorgegebene Umlaufzeit 4 ms, wird bei einer Transportgeschwindigkeit des Knüppels 1 von 45 - 60 m/min bzw. 0,75 - 1 mm/ms der Knüppel in Schritten von 3 - 4 mm abgetastet. Diese Schrittlänge reicht zur Analyse typischer mäanderförmiger Längsrisse 2 aus.

Der die einzelnen Sensorelemente aufnehmende Sensor 3 weist zweckmässigerweise einen Kühlwasserkanal auf, der zeichnerisch nicht dargestellt ist und eine Beschädigung der Sensorelemente durch die von dem heißen Knüppel 1 abgegebene große Wärme verhindert. Neben dem Kühlwasserkanal kann ein ebenfalls nicht gezeigter Druckluftkanal vorhanden sein, der mit mehreren Austrittsöffnungen in Verbindung steht. Die Austrittsöffnungen sind zur Beseitigung von Verschmutzungen vor Objektivfrontflächen angeordnet, die jedem Sensorelement zugeordnet sind.

Der beispielsweise vorgesehene Aluminiumring, dessen Außendurchmesser etwa 700 mm, dessen Innendurchmesser ungefähr 580 mm und dessen Breite ca. 100 mm betragen kann, besteht zweckmässigerweise aus zwei zusammengeschrumpften Aluminiumteilen, dessen Oberflächen vorzugsweise zwecks möglichst guter Strahlungsreflektion hochglänzend verchromt sind. Die Arbeitsentfernung zwischen den Sensorelementen und der Oberfläche eines Knüppels 1 von 220 mm Durchmesser beträgt bei dem oben angegebenen Knüppeldurchmesser 181,6 mm. Der Aluminiumring kann auch zur Aufnahme einer kompletten, kompakten Elektronik für die Sensorelemente ausgerüstet werden.

0105401
HH 82/22

Der optronische Sensor 3, der in einer Stranggußmaschine angeordnet wird, dient zusammen mit der nachgeschalteten Bildverarbeitungseinrichtung 8 bis 10 zur automatischen Erkennung
und Auswertung von Oberflächenfehlern an heißen Stranggrußprodukten, beispielsweise dem in den Figuren 1 und 2 dargestellten Knüppel 1. Hierzu werden die Produkte bzw. Knüppel 1
an dem Sensor 3 vorbeigeführt, der die Oberflächenfehler aufgrund der Temperaturunterschiede zu einer fehlerfreien Produktoberfläche bzw. zu fehlerfreien Produktoberflächenbereichen
meßtechnisch erfasst. Die Länge und Breite der Oberflächenfehler kann zu deren Quantisierung herangezogen werden. Auch ist
es möglich, die Größe der Oberflächenfehler zu klassifizieren,
die Lage der Oberflächenfehler, insbesondere ihre Koordinaten
in Bezug auf die zu kontrollierenden Produkte automatisch zu
ermitteln und auszuwerten, oder die Oberflächen bzw. die Oberflächenfehler auf einem Monitor 11 sichtbar darzustellen.
Hierbei kann ein lang nachleuchtender Monitor, oder ein Hardcopyrecorder Anwendung finden. Auch können einzelne Oberflächenzonen in einem Standbild oder in einem Rollbild auf dem
Monitor 11 dargestellt werden. Der Auftritt eines Oberflächenfehlers kann automatisch zum Einfrieren bestimmter Bilder ausgenutzt werden, die anschließend aberufen und auf dem Monitor
11 dargestellt werden. Zur Zwischenspeicherung von Bildern
kann ein Videorecorder verwendet werden, der bei Auftreten
eines Oberflächenfehlers ein Zurückspielen der letzten Bilder
gestattet. Weiterhin können von der Bildverarbeitungseinrichtung 8 bis 10 ermittelte statistische Daten in das Bild des
Monitors 11 eingeschrieben werden.

Die Einrichtung 9 dient unter anderem zur Auslösung eines
Alarmes, wenn die Oberflächenfehler vorgegebene Grenzwerte
hinsichtlich Anzahl oder Größe der Oberflächenfehler überschreiten. Die Alarmgabe kann z. B. über einen Relaiskontakt erfolgen, zu dem parallel eine Indikatorlampe angesteuert werden
kann.

Die Prüfung der zu kontrollierenden Produkte wird zweckmässigerweise nach dem Entzundern ihrer Oberflächen durchgeführt. Die Entzunderung kann mit Hilfe von Druckwasser erfolgen, wobei die Angriffsstelle des Druckwassers mit einer großen, eine Beschädigung der Produkte verhindernder Geschwindigkeit über die Produkte bewegt wird. Bei Stranggußknüppeln 1 wird die Entzunderung vorteilhafterweise beim schnellen Durchlauf der Produkte durch die Entzunderungsanlage durchgeführt, wobei die Produkte mit derselben Geschwindigkeit an dem Sensor 3 vorbeigeführt werden. Zur Qualitätskontrolle von Stranggußbrammen oder -vorblöcken erfolgt die Entzunderung am besten in der Stranggußmaschine, während die Oberflächenprüfung mit Hilfe des Sensors 3 dicht hinter der Entzunderung erfolgt.

Zur Auswertung von Oberflächenfehlern ist es erforderlich, die Geschwindigkeit und die Temperatur der zu kontrollierenden Produkte zu ermitteln. Hierzu kann der optronische Sensor 3 ebenfalls herangezogen werden.

Das Ausgangssignal des Sensors 3, bei dem es sich um ein serielles Signal der Sensorelemente handelt, wird der Einrichtung 6 zur Harmonisierung zugeführt. Es wird einer additiven und multiplikativen Korrektur unterzogen, um Dunkelstrom- und Empfindlichkeitsunterschiede der beispielsweise 4096 Fotodioden auszugleichen, so daß bei gleichmässig strahlender Oberfläche eines Knüppels 1 ein optimal gleichmässiges Bildsignal entsteht. Zum Ausgleich der Dunkelstrom- und Empfindlichkeitsunterschiede können zwei löschbare Read-Only-Memory-Bauelemente (EPROM's) mit einer Anzahl der Sensorelemente entsprechenden Anzahl von Plätzen vorgesehen sein, die zur Abspeicherung von mit 8 bit quantisierten Korrektursignalen für einen additiven bzw. multiplikativen Signalausgleich der Dunkelspannungs- bzw. Empfindlichkeitsschwankungen jedes einzelnen Sensorelementes dienen. Die EPROM's sind zu den Sensorelementen synchron auslesbar. Ihre digitalen Ausgangssignale können mit Hilfe

eines Digital-Analogwandlers in Analogsignale umgewandelt werden. Die analogen additiven Korrektursignale werden mittels analoger Addierglieder zu den Ausgangssignalen der Sensorelemente addiert; eine multiplikative Korrektur der Ausgangssignale der Sensorelemente erfolgt mittels analoger Multiplizierer.

Zur Vermeidung von Überbelichtungen an den Sensorelementen ist eine Belichtungsautomatik vorgesehen, die ein Überschreiten eines vorgegebenen Wertes der Ausgangsspannung des Sensors 3 verhindert. Der vorgegebene Wert kann beispielsweise bei 0,8 V liegen. Bei höheren Temperaturen wird die Integrationszeit der Sensorelemente mittels einer Regeleinrichtung automatisch derart geregelt, daß der vorgegebene Wert der Ausgangsspannung des Sensors nicht überschritten wird. Die Regelzeitkonstante der Regeleinrichtung ist derart wählbar, daß bei einem plötzlichen Signalsprung am Anfang des zu kontrollierenden Produktes nach einer Umlaufzeit von etwa 40 ms der eingeschwungene Zustand erreicht wird.

Zur Auswertung der vom Sensor 3 erkannten Oberflächenfehler wird vorzugsweise eine digitale Bildverarbeitungseinrichtung 8 bis 10 verwendet. Von dieser Einrichtung werden nur die, beispielsweise von Rissen erzeugten schmalen positiven oder negativen Impulse detektiert, während breite, Zunderresten zugeordnete Signale unterdrückt werden. Ein detektierter schmaler Impuls wird nur dann zur Anzeige gebracht, wenn bei den nächsten Umläufen in der engen Nachbarschaft des ersten Impulses weitere festgestellt werden. Es werden keine punkt- oder flächenförmigen, sondern nur in Transportrichtung des Knüppels 1 ausgedehnte Störungen angezeigt, d. h. die digitale Bildverarbeitungseinrichtung 8 bis 10 kann große, Zunderreste darstellende Objekte bereits in Echtzeit unterdrücken.

Die digitale Bildverarbeitungseinrichtung kann mehrere Oberflächenfehler pro Umlauf analysieren. Hierbei besteht das binärisierte Bild einer Knüppeloberfläche bei einer Transportgeschwindigkeit von 1 mm/ms und einer elektronischen Umlaufzeit von 4 ms aus jeweils 4096 Bildelementen pro Umfang, wobei die einzelnen Umläufe in Transportrichtung einen Abstand von 4 mm aufweisen. Bei einem mittleren Knüppelumfang von $\pi \cdot 197$ mm hat ein Bildelement die Querdimension $d = \pi \cdot 197/4096 = 0,15$ mm. Die auf die Umlaufzeit bezogene, für die weiteren Betrachtungen benutzte Längsdimension beträgt $l = 4$ mm, wobei die wahre Länge bei Belichtungszeiten, die kürzer als die Umlaufzeit sind, auch entsprechend kürzer sein kann. Nach den bisher vorliegenden Erkenntnissen sind zu Qualitätsverlusten führende Risse, die erkannt werden müssen, länger als 5 $l$ und zwischen 1 d und 15 d breit. Etwaige, nach der Entzunderung zurückgebliebene Zunderplättchen dagegen haben eine näherungsweise quadratische Struktur. Wenn sie also z. B. 10 d breit sind, liegt ihre Länge unter 1 $l$; wenn sie länger als 5 $l$ sind, sind sie auch wesentlich breiter als 15 d. Bei der Ermittlung von Rißlängen ist deren typischer mäanderförmiger Verlauf mit Auslenkungen um etwa $\pm$ 32 d sowie das Auftreten von Unterbrechungen von 1 - 2 $l$ zu beachten. Diese Unterbrechungen können durch Verengungen eines Risses verursacht sein.

Bei der Analyse von mehreren Oberflächenfehlern pro Umlauf kann eine Mikroprozessorsoftware mehrere relevante Oberflächenfehler aus verschiedenen Umläufen verwalten und zwecks Längen- und Breitenschätzung bei jedem neuen Umlauf daraufhin untersuchen, ob die aktuellen Oberflächenfehler mit dem bereits verwalteten Oberflächenfehler in Zusammenhang stehen. Zur Analyse schräger Risse in jedem Umlauf kann die digitale Bildverarbeitungseinrichtung in jedem Umlauf ein vom letzten zugehörigen Oberflächenfehler positioniertes Erwartungs-Suchbereichsfenster setzen. Zur Detektion von Sternchenrissen kann die Mikroprozessorsoftware eine Anhäufung von kurzen Rissen analysieren (Clusterung).

Zur Detektion anderer optischer Oberflächenfehler kann die digitale Bildverarbeitungseinrichtung 8 bis 10 mit einem Gradientenbildprozessor ausgerüstet sein; oder es kann zur Detektion von optischen Oberflächeneffekten eine Einrichtung zur Grauwertbildverarbeitung vorgesehen sein, die eine Bildpunktmengenanalyse der Oberflächeneffekte über Mittelwert, Varianz oder lokaladaptive Schwellwertberechnung durchführt. In diesem Falle setzt die Bildverarbeitung direkt hinter der Harmonisierungsstufe 6 ein.

Zur Prüfung der Sensorelemente des Sensors 3 kann ein gleichmässig diffus strahlender Zylinder in den Sensor 3 als Eichquelle eingebracht werden. Um die Sensorprüfung durchzuführen, kann die Elektronikbox 5 eine zeichnerisch nicht dargestellte Auswerteschaltungsanordnung aufweisen, die durch eine Prüftaste aktivierbar ist und die den Spitze/Spitze-Wert und den Gleichstromanteil des Ausgangssignals mißt bzw. prüft. Weiterhin kann die Elektronikbox 5 mit einer Indikatorlampe versehen sein, die bei einer Sensorprüfung dann aufleuchtet, wenn die Sensorelemente in Ordnung sind. Auch ist es möglich, die Prüfvorrichtung mit einem programmierten Read-Only-Memory-Bauelement (PROM) auszustatten, das typische Oberflächenstrukturen enthält und über eine Prüftaste anstelle der Sensorelemente auf die Bildverarbeitungseinrichtung schaltbar ist.

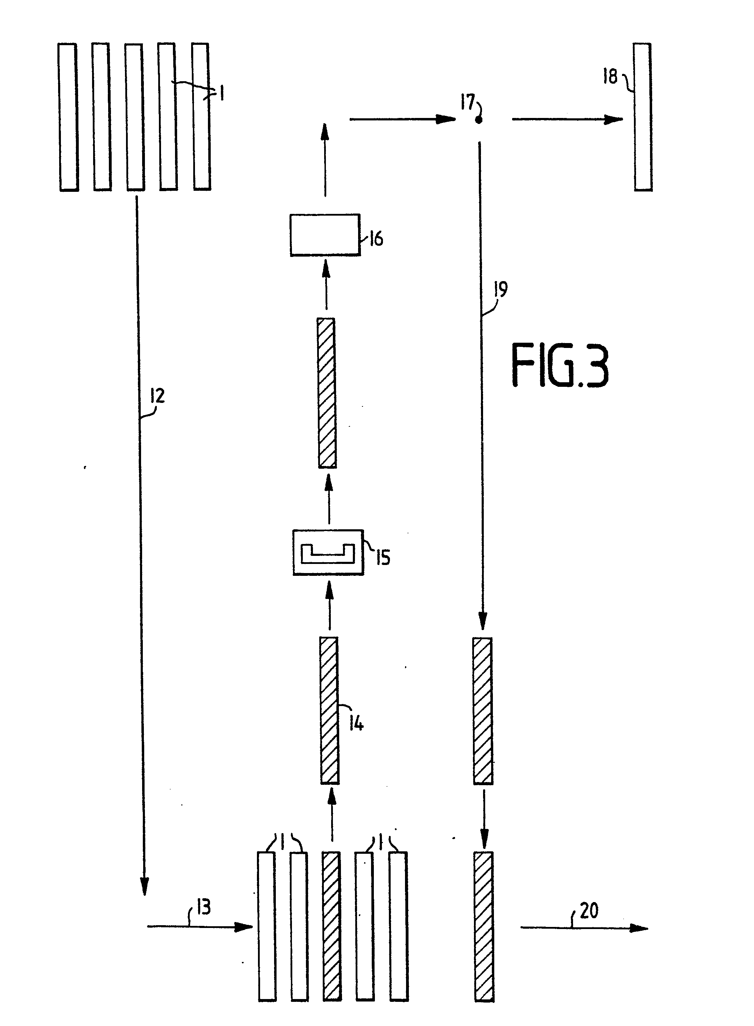

Gemäß dem in Figur 3 dargestellten Verfahrensablauf werden die aus einer zeichnerisch nicht dargestellten Gießmaschine kommenden Knüppel 1 einem Rollgang 12 mit Brennschneidmaschinen zugeführt. Die Brennschneidmaschinen sind ebenfalls nicht gezeichnet. Nach dem Schneiden der Knüppel auf eine vorgegebene Länge erfolgt ein Quertransport der Knüppel 1, dessen Richtung durch den Pfeil 13 gekennzeichnet ist. Durch den Quertransport werden die Knüppel 1 direkt zu einer nicht gezeigten Einrichtung 20 zur Weiterverarbeitung befördert, z. B. zu einem einem Walzwerk vorgeschalteten Ofen.

Aus dem Quertransport können nach vorgegebenen Auswahlkriterien die Knüppel 1 einer Inspektionslinie 14 zugeführt werden. Hier durchlaufen sie zuerst eine Entzunderungsanlage 15 zur Entzunderung der Oberflächen. Bei einer Entzunderung mit Hochdruckwasser muß die Entzunderungsgeschwindigkeit so groß sein, daß das Stranggußprodukt nicht beschädigt wird.

Unmittelbar nach der Entzunderungsanlage 15 ist ein optisches System 16 angeordnet, das Oberflächenfehler 2 am Stranggußprodukt 1 trotz hoher Durchlaufgeschwindigkeit (bis 2 m/s) und hoher Temperaturen (bis 1000° C) erkennt. Hierbei kann es sich um die in Fig. 2 dargestellte Vorrichtung handeln, dessen Sensor 3 die Knüppel 1 vollständig umgibt und dessen zugehörende Elektronikbox 5 mit nachgeordnetem Monitor 11 von dem Sensor 3 räumlich entfernt angeordnet ist.

Nach Vergleich der detektierten Qualität, die die Qualitätsstelle eines Hüttenwerkes vorgibt, wird sofort an dem Ort 17 der Anlage die Entscheidung getroffen, ob der Knüppel 1 zur weiteren Inspektion und Nacharbeit einem Sonderrollgang 18 zugeführt wird oder ob der Knüppel 1 die Linie 19 durchläuft und zur weiteren Verarbeitung in der Einrichtung 20 freigegeben wird.

Bei der erfindungsgemäßen Vorrichtung ist zur Erhöhung der Empfindlichkeit die Verwendung einer Bildverstärkerröhre bzw. einer Bildwandlerröhre, z.B. einer EB-CCD-Sensorkombination im nahen IR-Spektralbereich möglich. Auch können zur Abschattung der Wärmestrahlung Schattenbleche vorgesehen sein.

Zur Ermittlung der Tiefe eines Risses kann eine entsprechende Einrichtung der Bildverarbeitungseinrichtung nachgeschaltet sein. Diese Einrichtung kann nach einem Wirbelstrommeßverfahren arbeiten, wobei dieses Wirbelstrommeßverfahren je nach dem erkannten Längs- oder Querriß in Längs- oder Querrichtung angewendet werden kann. Auch kann die endgültige Ablieferung
eines von der Bildverarbeitungseinrichtung ermittelten Rißalarmes durch das Wirbelstrommeßverfahren mitbestimmt werden.

05  Zur Anwendung können CCD-Zeilen-Sensorkombinationen vom Typ
    Reticon CCPD 256, CCPD 512, CCPD 1024 oder CCPD 1728 kommen.

Licentia Patent-Verwaltungs-GmbH          PTL-HH/Sl/mar
Theodor-Stern-Kai 1                       HH 82/22
D-6000 Frankfurt 70
und
MANNESMANN AKTIENGESELLSCHAFT
Mannesmannufer 2
D-4000 Düsseldorf 1


PATENTANSPRÜCHE


1. Verfahren zur Qualitätsbeurteilung von Stahloberflächen, insbesondere zur Erkennung von Oberflächenfehlern, wie Rissen an heißen, entzunderten Stranggußprodukten, wobei in einer Stranggußmaschine ein optronischer Sensor angeordnet ist, an dem die zu kontrollierenden Produkte vorbeigeführt werden, der die von den Produkten emittierte Oberflächenstrahlung empfängt und in auswertbare Signale umsetzt, und der mit einer nachgeschalteten Verarbeitungseinrichtung zur automatischen Erkennung und Auswertung der Oberflächenfehler verwendet wird, dadurch gekennzeichnet, daß eine elektronische Kamera mit elektronischer Kurzzeitbelichtung vorgesehen ist, und daß eine digitale Verarbeitungseinrichtung nach vorgegebenen Längen- und Breitenkriterien der Oberflächenfehler zur Qualitätsbeurteilung herangezogen wird.


2. Verfahren nach Anspruch 1, dadurch gekennzeichnet, daß die Geschwindigkeit und die Temperatur der zu kontrollierenden Produkte (1) mittels Silizium-Fotodioden ermittelt werden, und daß die ermittelten Ergebnisse zur Auswertung der Oberflächenfehler (2) verwendet werden.

3. Verfahren nach Anspruch 1, dadurch gekennzeichnet, daß zur Ermittlung der Geschwindigkeit und der Temperatur der zu kontrollierenden Produkte (1) der optronische Sensor (3) herangezogen wird, und daß die ermittelten Ergebnisse zur Auswertung der Oberflächenfehler (2) verwendet werden.

4. Verfahren nach Anspruch 1, 2 oder 3, dadurch gekennzeichnet, daß eine Bildspeichereinrichtung (10) verwendet wird, und daß ein Auftritt eines Oberflächenfehlers (2) automatisch zum Einfrieren bestimmter Bilder ausgenutzt wird, die anschließend abgerufen und auf einem Monitor (11) dargestellt werden.

5. Vorrichtung zur Durchführung des Verfahrens nach einem der Ansprüche 1 bis 4, dadurch gekennzeichnet, daß der optronische Sensor (3) eine vorgegebene Anzahl von Sensorelementen aufweist, die die zu kontrollierenden Produkte (1) vollständig umgeben, daß jedes Sensorelement einen bestimmten Sektor der Produkte (1) abtastet, und daß der Sensor (3) seine Bildsignale an die Bildverarbeitungseinrichtung (8 - 10) abgibt.

6. Vorrichtung nach Anspruch 5, gekennzeichnet durch eine Halbleiter-Zeilen-Sensorkombination.

7. Vorrichtung nach Anspruch 5, gekennzeichnet durch eine elektronische Kurzzeitbelichtungskamera auf Röhren-Sensorbasis mit getasteter Hochspannung.

8. Vorrichtung nach Anspruch 5, gekennzeichnet durch eine Kurzzeitbelichtungskamera auf Halbleiter-Matrix-Sensorbasis.

9. Vorrichtung nach einem der Ansprüche 5 bis 8, dadurch gekennzeichnet, daß die Sensorelemente mittels eines Kühlwasserkanals gekühlt werden.

10. Vorrichtung nach einem der Ansprüche 5 bis 9, dadurch gekennzeichnet, daß ein Druckluftkanal vorgesehen ist, der mit mehreren Austrittsöffnungen in Verbindung steht, und daß die Austrittsöffnungen zur Beseitigung von Verschmutzungen vor den Objektivfrontflächen der Sensorelemente angeordnet sind.

11. Vorrichtung nach einem der Ansprüche 5, 6, 8, 9 oder 10, dadurch gekennzeichnet, daß zum Ausgleich von Dunkelstrom- und Empfindlichkeitsunterschieden der Sensorelemente und zur Erzeugung eines optimal gleichmäßigen Bildsignals bei gleichmässig strahlender Stahloberfläche zwei löschbare Speicher-Bauelemente (RAM's, EPROM's) mit einer der Anzahl der Sensorelemente entsprechenden Anzahl von Plätzen vorgesehen sind, die zur Abspeicherung von mit 8 bit quantisierten Korrektursignalen für einen additiven bzw. multiplikativen Signalausgleich der Dunkelstrom- bzw. Empfindlichkeitsschwankungen jedes einzelnen Sensorelementes dienen.

12. Vorrichtung nach Anspruch 5 oder 6, dadurch gekennzeichnet, daß eine Regeleinrichtung vorgesehen ist, die bei konstanter Auslesefrequenz die Integrationszeit der Sensorelemente bei höheren Temperaturen automatisch derart regelt, daß der vorgegebene Wert der Ausgangsspannung des Sensors (3) nicht überschritten wird.

13. Vorrichtung nach Anspruch 5, dadurch gekennzeichnet, daß zur Detektion von optischen Oberflächeneffekten eine Einrichtung zur Grauwertbildverarbeitung vorgesehen ist, die mittels Matrixoperationen eine Bildpunktmengenanalyse der Oberflächeneffekte über Mittelwert, Varianz oder lokaladaptive Schwellwertberechnung durchführt.

# FIG.1

# FIG.2

0105401

FIG.3